# EUROPEAN PATENT SPECIFICATION

(11) **EP 3 310 328 B1**
(45) Date of publication and mention of the grant of the patent: **20.05.2020**
(21) Application number: 16811009.6
(22) Date of filing: 16.06.2016
(51) Int. Cl.: A61K 8/34, A61Q 19/00, A61Q 90/00, A61K 8/44, A61K 8/06, A61K 8/31, A61K 8/46, A61K 8/49

(54) **EMULSION COMPOSITION HAVING HIGH CONTENT OF OIL**
EMULSIONSZUSAMMENSETZUNG MIT HOHEM ÖLGEHALT
COMPOSITION D'ÉMULSION PRÉSENTANT UNE TENEUR ÉLEVÉE EN HUILE

(30) Priority: 18.06.2015 WO PCT/CN2015/081755
(43) Date of publication of application: 25.04.2018
(73) Proprietor: Rhodia Operations, 75009 Paris (FR)
(72) Inventor: ZHANG, Hai Zhou, Singapore 128803 (SG); SONG, Qing Rong, Shanghai 200240 (CN); HE, Lin, Singapore 357903 (SG); LIM, Lynlie, Singapore 680641 (SG)
(74) Representative: Cardon, Flavie
(86) International application number: PCT/CN2016/085960
(87) International publication number: WO 2016/202268

(56) References cited:
- EP-A2- 0 870 495
- WO-A1-2009/080005
- WO-A1-2014/020145
- WO-A1-2014/098254
- WO-A1-2015/054135
- DE-A1-102010 029 628
- US-A1- 2011 232 667
- US-A1- 2014 194 535

## Description

### Technical Field

The present invention relates to an emulsion composition, in particular, an emulsion composition comprising high content of oil, a polyol and an amphoteric surfactant. The emulsion composition may be used for personal care applications, pharmaceutical applications or cosmetic applications.

### Background Art

Emulsion compositions are widely used for personal care, pharmaceutical or cosmetic products, such as cleaning foams and gels, body lotions and milks, makeup oils, massage oils and oils having therapeutical properties. It is desired that such emulsion compositions have excellent stability, appropriate viscosity, and good transparency as well. It is also advantageous that such emulsion compositions have high oil content because high oil content can provide special skin feel and good spreadability, and it can be helpful for the fast release of the internal oil phase or active ingredients/perfumes contained in the emulsion internal phase as well.

Generally, the emulsion compositions as mentioned above contain combination of a hydrophobic oil phase and a hydrophilic phase, such as water and alcohol. In order to provide such emulsion compositions with good stability, surfactants are often included in the composition as emulsifiers or stabilizers. Conventionally, nonionic surfactants have been used for such purposes and the nonionic surfactants mostly used are ethoxylated nonionic surfactants. For example, it is disclosed in US publication no. US 5474776A that nonionic surfactants can be used as emulsifiers for oil-in-water emulsions. However, there is a concern that ethoxylated surfactants may be hazardous to the environment, in particular, when such surfactants are used at high amounts. WO2014/020145 discloses a dye composition, which is in cream form and which comprises at least a dye precursor, an ionic surfactant, and oil and a thickening agent.

Due to the complexity of emulsion systems, it remains a challenge to provide an emulsion composition which can meet the requirements of the desired application. There is a need to provide an emulsion composition which has excellent stability. There is also a need to provide a stable emulsion composition with high oil content. There is further a need to provide an emulsion composition which requires minimal amount of surfactant for maintaining the stability of the compositions.

### Summary of Invention

It has been found that the above objectives can be achieved by the present invention.

In one aspect of the present invention, there is provided an emulsion composition comprising :
(a) from 65 wt% to 90 wt% of an oil phase;
(b) from 5 wt% to 30 wt% of a polyol;
(c) from 0.01 wt% to 7.5 wt% of an amphoteric surfactant;
(d) from 0 wt% to 0.5 wt% of a nonionic surfactant;
percent by weight is based on the total weight of the composition.

Preferably, the composition comprises :
(a) from 70 wt% to 90 wt% of an oil phase;
(b) from 5 wt% to 25 wt% of a polyol;
(c) from 0.01 to 7.5 wt% of an amphoteric surfactant;
(d) from 0 wt% to 0.5 wt% of a nonionic surfactant;
percent by weight is based on the total weight of the composition.
More preferably, the composition comprises :
(a) from 70 wt% to 90 wt% of an oil phase;
(b) from 5 wt% to 25 wt% of a polyol;
(c) from 0.01 to 5 wt% of an amphoteric surfactant;
(d) from 0 wt% to 0.5 wt% of a nonionic surfactant;
percent by weight is based on the total weight of the composition.

Preferably, the emulsion composition is substantially free or completely free of nonionic surfactant.

The amphoteric surfactant may be chosen from the group consisting of:
betaine, amine oxide, amphoglycinate and amphopropionate.

Advantageously, the amphoteric surfactant is a compound having the general formula of :

**R₁ R₂ R₃ N⁺ (CH₂)_{y} C(O)O⁻** (I)

or

**(R₁)(R₂C(O)NH(CH₂)ₓ)(R₃)N⁺(CH₂)_{y} C(O)O⁻** **(II)**

wherein R₁ is a C₁-C₅ alkyl or alkenyl group which is optionally hydroxylated;
R2 is a C₁₀-C₂₂-alkyl or C₁₀-C₂₂-alkenyl group;
R₃ is independently selected from a C₁-C₅ alkyl or alkenyl group, C₁₀-C₂₂-alkyl or C₁₀-C₂₂-alkenyl group;
x is an integer of 2-4, y is an integer of 1-4.

In one preferred embodiment, the amphoteric surfactant is a compound having the general formula of:

**R₇-N⁺(CH₃)₂-CH₂-COO⁻** **(IX)**

wherein R₇ is a C₁₀-C₂₂-alkyl or C₁₀-C₂₂-alkenyl group.

Advantageously, the amphoteric surfactant is a compound having the general formula of :

**R₁ R₂ R₃ N⁺R₄-SO₃⁻** **(III)**

or

**(R₁)(R₂C(O)NH(CH₂)ₓ)(R₃)N⁺R₄-SO₃⁻** **(IV)**

wherein R₁ is a C₁-C₅ alkyl or alkenyl group which is optionally hydroxylated;
R2 is a C₁₀-C₂₂-alkyl or C₁₀-C₂₂-alkenyl group;
R₃ is independently selected from a C₁-C₅ alkyl or alkenyl group, C₁₀-C₂₂-alkyl or C₁₀-C₂₂-alkenyl group;
R₄ is a C₁-C₄ alkyl group, linear or branched, optionally hydroxylated;
x is an integer of 2-4.

Advantageously, the amphoteric surfactant is a compound having the general formula of :

**R₁ R₂ R₃ N⁺ --O⁻** **(V)**

or

**(R₁)(R₂C(O)NH(CH₂)ₓ)(R₃)N⁺--O⁻** **(VI)**

wherein R₁ is a C₁-C₅ alkyl or alkenyl group which is optionally hydroxylated;
R2 is a C₁₀-C₂₂-alkyl or C₁₀-C₂₂-alkenyl group;
R₃ is independently selected from a C₁-C₅ alkyl or alkenyl group, C₁₀-C₂₂-alkyl or C₁₀-C₂₂-alkenyl group;
x is an integer of 2-4.

Advantageously, the amphoteric surfactant is a compound having the general formula of :

**R₂ N(R₅)(CH₂)_{z} C(O)O⁻ Y⁺** **(VII)**

or

**R₂ C(O)N(R₅)(CH₂)ₓ N(R₆)(CH₂)_{z} C(O)O⁻ Y⁺** **(VIII)**

wherein R₂ is a C₁₀-C₂₂-alkyl or C₁₀-C₂₂-alkenyl group;
R₅ is hydrogen or a C₁-C₅ group which is optionally hydroxylated;
R₆ is a C₁-C₅ group which is optionally hydroxylated or a (CH₂)_{z} C(O)O⁻ group;
z is an integer of 1-4;
x is an integer of 2-4;
**Y⁺** is a cation.

The polyol may be a compound having the formula :

**R₈-C(-R₉)(-OH)-[A-C(-OH)(R₁₀)]ₙ-R₁₁** **(X)**

wherein
R₈, R₉, R₁₀ and R₁₁ are each independently selected from the group comprising: hydrogen, C₁-C₆ hydrocarbyl, C₁-C₆ cycloalkyl and C₁-C₆ heterocyclic moiety; said hydrocarbyl moiety is saturated or unsaturated, branched or linear; each of said hydrocarbyl, cycloalkyl and heterocyclic moiety is optionally substituted with one or several substituents;
A is a covalent bond or a linking moiety;
n is an integer of 1 to 10.

Advantageously, the polyol is selected from propylene glycol, isoprene glycol, 1,3-butanediol, dipropylene glycol, glycerol, diglycerol, triglycerol, polyglycerol, trimethylolpropane, erythritol, pentaerythritol, sorbitan, sorbitol, glucose, maltitol, saccharose, trehalose, polyethylene glycol and a mixture thereof.

### Detailed Description

Throughout the description, including the claims, the term "comprising one" or "comprising a" should be understood as being synonymous with the term "comprising at least one", unless otherwise specified, "between" and "from... to..." should be understood as being inclusive of the limits.

As used herein, "weight percent," "wt%," "percent by weight," "% by weight," and variations thereof refer to the concentration of a substance as the weight of that substance divided by the total weight of the composition and multiplied by 100.

It should be noted that in specifying any range of concentration, amount or ratio, any particular upper concentration, amount or ratio can be associated with any particular lower concentration, amount or ratio, respectively.

"Alkyl" as used herein means a straight chain or branched saturated aliphatic hydrocarbon group and is intended to include both "unsubstituted alkyl" and "substituted alkyl", the latter of which refers to alkyl moieties having substituents (such as hydroxyl group and halogen group) replacing a hydrogen on one or more carbon atoms of the alkyl group. "Alkenyl", as used herein, refers to an aliphatic group containing at least one double bond and is intended to include both "unsubstituted alkenyls" and " substituted alkenyls", the latter of which refers to alkenyl moieties having substituents (such as hydroxyl group and halogen group) replacing a hydrogen on one or more carbon atoms of the alkenyl group.

The present invention relates to an emulsion composition comprising :
(a) from 65 wt% to 90 wt% of an oil phase;
(b) from 5 wt% to 30 wt% of a polyol;
(c) from 0.01 wt% to 7.5 wt% of an amphoteric surfactant;
(d) from 0 wt% to 0.5 wt% of a nonionic surfactant;
percent by weight is based on the total weight of the composition.

Notably, the composition comprises :
(a) from 70 wt% to 90 wt% of an oil phase;
(b) from 5 wt% to 25 wt% of a polyol;
(c) from 0.01 to 7.5 wt% of an amphoteric surfactant;
(d) from 0 wt% to 0.5 wt% of a nonionic surfactant;
percent by weight is based on the total weight of the composition.

Preferably, the emulsion composition of the present invention is substantially free or completely free of any nonionic surfactant.

The oil phase suitable for the present invention can be emollient oils which are generally used for personal care or cosmetic products. The oil phase is generally an oily material that is liquid at ambient temperature (25 °C). Alternatively, the oils may be solid at the ambient temperature which is provided in the form of waxy solid and which can become liquid at an elevated temperature at which it can be included and emulsified in the composition of the present invention. When oils which are solid at the ambient temperature are used, the oils preferably have melting temperature of from -30 °C to 100 °C, more preferably, have melting temperature of from -30 °C to 70 °C.

Suitable liquid oils include non-polar oils, for example mineral or paraffin, especially isoparaffin oils, such as that sold by ICI Surfactants as Arlamol HD, or medium polarity oils, for example vegetable glyceride oils such as jojoba oil, animal glyceride oils, such as that sold by ICI Surfactants as Arlamol M812 (caprylic/capric triglyceride), synthetic oils, for example synthetic ester oils such isopropyl palmitate and those sold by ICI Surfactants as Arlamol IPM and Arlamol DOA, ether oils, in particularly those of two fatty, e. g. Cs to C₁₈ alkyl residues, such as that sold by Henkel as Eutanol G (octyl codecanol) or silicone oils such as dimethicione oil such as those sold by Dow Corning as DC200 cyclomethicone oil, or silicones having polyoxyalkylene side chains to improve their hydrophilicity, or highly polar oils including alkoxylate emollients for example fatty alcohol propoxylates such as that sold by ICI Surfactants as Arlamol E (stearyl alcohol 15-propoxylate). Suitable oils also include white mineral oils and dicaprylyl carbonate. Suitable oils that can be solid at ambient temperature but liquid at temperatures typically used to make the compositions of this invention include jojoba wax, tallow and coconut wax/oil.

According to the present invention, mixtures of oils can be used. In some cases, solid oils may dissolve wholly or partly in liquid oils provided that the freezing point of the resulting mixture is suitably low. Where the oil is a solid at the ambient temperature, the resulting dispersion may technically not be an emulsion. However, such dispersion behaves as if it was true emulsion, and therefore, the term emulsion is used herein to include such composition.

According to the present invention, the content of the oil phase is in the range of 65 wt% to 90 wt%, preferably, in the range of 70 wt% to 90 wt%, more preferably in the range of 70 wt% to 85 wt%.

In the context of the present invention, the term amphoteric surfactant refers to a compound having both cationic and anionic centers attached to the same molecule. In particular, the cationic part is based on primary, secondary, or tertiary amines or quaternary ammonium cations. The anionic part includes, but is not limited to, carboxylate, sulfonate and phosphate. More in particular, the amphoteric surfactant refers to compounds having an N⁺-O⁻ function, a quaternary N⁺ function in combination with a C(O)O⁻, SO₃H or SO₃⁻ function, and to compounds having a tertiary N function in combination with a C(O)OH, C(O)O⁻, SO₃H or SO₃⁻ function.

For an overview of amphoteric surfactants and their properties the reader is referred to Amphoteric Surfactants, 2nd ed., E. G. Lomax, Ed., 1996, Marcel Dekker. This class of surfactants includes betaines, e.g., fatty alkyl betaines, fatty alkylamido betaines, sulfobetaines, hydroxysulfobetaines, and betaines derived from imidazolines; amine oxides, e.g., fatty alkylamine oxides and fatty alkylamido amine oxides; amphoglycinates and amphopropionates; and so-called "balanced" amphopoly-carboxyglycinates and amphopolycarboxypropionates.

Amphoteric surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents contains a C₁₀-C₂₂-alkyl or C₁₀-C₂₂-alkenyl group, preferably, a C₁₆-C₂₂-alkyl or C₁₆-C₂₂-alkenyl group.

The amphoteric surfactants of the present invention may notably be chosen from the group consisting of: betaine, amine oxide, amphoglycinate and amphopropionate.

Betaines are a class of amphoteric surfactants which include compounds having the structures:

**R₁ R₂ R₃ N⁺** (CH₂)_{y} **C(O)O⁻** **(I)**

or

**(R₁)(R₂C(O)NH(CH₂)ₓ)(R₃)N⁺(CH₂)yC(O)O⁻** **(II)**

wherein R₁ is a C₁-C₅ group which is optionally hydroxylated, such as a methyl, ethyl, hydroxyethyl, or hydroxypropyl group; R₂ is a C₁₀-C₂₂-alkyl or C₁₀-C₂₂-alkenyl group; R₃ is independently selected from a C₁-C₅ group or C₁₀-C₂₂-alkyl or C₁₀-C₂₂-alkenyl group as defined for R₁ and R₂, respectively; x is an integer of 2-4, y is an integer of 1-4; wherein any two of the groups R₁-R₃ optionally may form a ring structure. The C₁-C₅ group may be an alkyl or alkenyl group.

Preferably, R₂ is C₁₆-C₂₂-alkyl or C₁₆-C₂₂-alkenyl. Preferably, R₃ is independently selected from a C₁-C₅ group or C₁₆-C₂₂-alkyl or C₁₆-C₂₂-alkenyl group.

In the context of the present invention, betaine also include sulfobetaines and hydroxysulfobetaines which have structures according to (I) and (II), having R₁, R₂, and R₃ defined as above, wherein the group (CH₂)_{y}C(O)O⁻ has been replaced by a C₁₋₄-SO₃⁻ group, in which C₁-C₄ group is optionally hydroxylated. The compound may be illustrated by the general formula :

**R₁ R₂ R₃ N⁺R₄-SO₃⁻** **(III)**

or

**(R₁)(R₂C(O)NH(CH₂)ₓ)(R₃)N⁺R₄-SO₃-** **(IV)**

wherein R₄ is a C₁-C₄ alkyl group, linear or branched, optionally hydroxylated, x is as defined in Formula (II).

Amine oxides are a class of amphoteric surfactants which include compounds having the structure :

**R₁ R₂ R₃ N⁺ --O⁻** **(V)**

or

**(R₁)(R₂C(O)NH(CH₂)ₓ)(R₃)N⁺--O⁻** **(VI)**

wherein R₁, R₂, and R₃ and x have the meaning described above.

Amphoglycinates and amphopropionates are classes of amphoteric surfactants which include compounds having the structures:

**R₂ N(R₅)(CH₂)_{z} C(O)O⁻ Y⁺** **(VII)**

or

**R₂ C(O)N(R₅)(CH₂)ₓN(R₆)(CH₂)_{z}C(O)O⁻ Y⁺** **(VIII)**

wherein R₂ and x have the meaning described above; R₅ is hydrogen or a C₁-C₅ group which is optionally hydroxylated, R₆ is a C₁-C₅ group which is optionally hydroxylated or a (CH₂)_{z}C(O)O⁻ group, z is an integer of 1-4, and Y⁺ is a cation, such as a proton or a sodium ion.

It is appreciated that in the general formula (VII) and the general formula (VIII), the compound is an amphoglycinate when z=1, and the compound is an amphopropionate when z=2.

In particular, the amphoteric surfactant of the present invention is a compound of formula (IX):

**R₇-N⁺(CH₃)₂-CH₂-COO⁻** **(IX)**

wherein R₇ is C₁₀-C₂₂-alkyl or C₁₀-C₂₂-alkenyl group.

In the general formula (IX), preferably R₇ is C₁₆-C₂₂-alkyl, such as C₁₆-alkyl, C₁₈-alkyl, C₂₀-alkyl and C₂₂-alkyl. Or C₁₆-C₂₂-alkenyl, such as C₁₆-alkenyl, C₁₈-alkenyl, C₂₀-alkenyl and C₂₂-alkenyl.

In a preferred embodiment of the present invention, the compound of formula (IX) is chosen in the group constituted of: cetyl betaine, palmityl betaine and oleyl betaine.

According to the present invention, the emulsion composition comprises from 0.01 wt% to 7.5 wt% of the amphoteric surfactant, preferably from 0.01 wt% to 5 wt% of the amphoteric surfactant, more preferably from 0.01 wt% to 2 wt% of the amphoteric surfactant.

The polyol is an alcohol compound comprising at least 2 hydroxyl groups. Notably, the polyol may be represented by formula (X):

**R₈-C(-R₉)(-OH)-[A-C(-OH)(R₁₀)]ₙ-R₁₁** **(X)**

wherein
R₈, R₉, R₁₀ and R₁₁ are each independently selected from the group comprising: hydrogen, substituted or unsubstituted, branched or linear hydrocarbyl moieties; and substituted or unsubstituted carbocyclyl or heterocyclic moieties;
A is a covalent bond or a linking moiety;
n is an integer of 1 to 10, preferably from 1 to 5.

For example, each R₈, R₉, R₁₀ and R₁₁ may be independently selected from the group comprising C₁-C₆ alkyl, C₁-C₆ cycloalkyl and C₁-C₆ heterocyclic moiety which may be saturated or unsaturated, wherein said alkyl moiety may be branched or linear, and said alkyl, cycloalkyl and heterocyclic moiety is optionally substituted with one or several substituents.

The linking moiety A may be any diradical. The linking moiety A may comprise substituted or unsubstituted hydrocarbylene or substituted or unsubstituted monocyclic or polycyclic carbocyclylene or heterocyclylene, and optionally one or several functional groups, such as ether function.

The term "hydrocarbyl" as used herein refers to a moiety consisting exclusively of carbon and hydrogen atoms. As defined herein, the hydrocarbyl moiety is branched or linear and is aliphatic. The hydrocarbyl moiety may contain one or several unsaturations, i.e. one or several double bonds or one or several triple bonds, or both. The moiety preferably comprises 1 to 6 carbon atoms.

Polyols suitable for the present invention include dialcohols, polyhydric alcohols, monosaccharides and disaccharides. Preferred polyols include propylene glycol, isoprene glycol, 1,3-butanediol, dipropylene glycol, glycerol, diglycerol, triglycerol, polyglycerol, trimethylolpropane, erythritol, pentaerythritol, sorbitan, sorbitol, glucose, maltitol, saccharose, trehalose, polyethylene glycol, with glycerol, sorbitol, maltitol being particularly preferred.

Polyol compounds may be used either singly or in any combination thereof for the present invention. The polyol is incorporated in the emulsion composition in the range of 5 wt% to 30 wt%, preferably in the range of 5 wt% to 25 wt%, more preferably in the range of 10 wt% to 25 wt% based on the total weight of the emulsion composition.

One objective of the present invention is to minimize the amount of nonionic surfactant in the emulsion composition. The emulsion composition may comprise from 0 wt% to 0.5 wt% of nonionic surfactant, based on the total weight of the emulsion composition. Preferably, the emulsion composition of the present invention is substantially free or completely free of any nonionic surfactant. As used herein, the term "substantially free" when used with reference to the absence of nonionic surfactant in the composition of the present invention, means that the composition comprises less than 0.1 wt % of the nonionic surfactant, more preferably less than 0.01 wt % of the nonionic surfactant, based on the total weight of the composition. As used herein, the term "completely free" when used with reference to the absence of the nonionic surfactant (i.e., 0 wt% of the anionic agent) in the composition of the present invention, means that the composition comprises no nonionic surfactant at all.

The emulsion composition of the present invention may additionally comprise water. The amount of water may be in the range of 0.01 wt% to 8 wt% based on the total weight of the composition, preferably in the range of 0.01 wt% to 5 wt% based on the total weight of the composition, more preferably in the range of 0.01 wt% to 2 wt% based on the total weight of the composition. In some aspects, the emulsion composition is substantially free of water. As used herein, the term "substantially free" when used with reference to the absence of water in the composition of the present invention, means that the composition comprises less than 0.1 wt % of the water, more preferably less than 0.01 wt % of the water, based on the total weight of the composition.

It is appreciated that components which are conventionally used in cosmetic compositions, pharmaceutical compositions or personal care compositions may be added to the emulsion composition of the present invention. Examples include medicinally-effective ingredients, moisturizing components, antiphlogistic agents, anionic surfactants, disinfectants, antiseptics, ultraviolet absorbents, antioxidants, organic and inorganic powders, colorants and perfumes, alone or in combination as needed.

### Perfumes

Referring to perfumes, perfumes suitable for the present invention include those that are natural, semi-synthetic or synthetic in origin. Preferred perfumes may be assigned to the classes of substance comprising the hydrocarbons, aldehydes or esters. The perfumes also include natural extracts and/or essences, which may comprise complex mixtures of constituents, i.e. fruits such as almond, apple, cherry, grape, pear, pineapple, orange, lemon, strawberry, raspberry and the like; musk, flower scents such as lavender, jasmine, lily, magnolia, rose, iris, carnation and the like; herbal scents such as rosemary, thyme, sage and the like; woodland scents such as pine, spruce, cedar and the like.

Other suitable perfumes are essential oils, resinoids and resins from a large number of sources, such as, Peru balsam, olibanum resinoid, styrax, labdanum resin, nutmeg, cassia oil, benzoin resin, coriander, clary sage, eucalyptus, geranium, lavender, mace extract, neroli, nutmeg, spearmint, sweet violet leaf, valerian and lavandin.

Some or all of the perfumes may be encapsulated, typical perfume components which it is advantageous to encapsulate, include those with a relatively low boiling point. It is also advantageous to encapsulate perfume components which have a low Clog P (i.e. those which will be partitioned into water), preferably with a Clog P of less than 3.0. As used herein, the term "Clog P" means the calculated logarithm to base 10 of the octanol/water partition coefficient (P).

Further suitable perfumes include: phenylethyl alcohol, terpineol, linalool, linalyl acetate, geraniol, nerol, 2-(1,1-dimethylethyl)cyclo-hexanol acetate, benzyl acetate, and eugenol.

### Essential Oils

Essential oils suitable for the present invention include oils derived from herbs, flowers, trees, and other plants. Non-limiting examples of essential oils include sesame oil, macadamia nut oil, tea tree oil, evening primrose oil, Spanish sage oil, Spanish rosemary oil, coriander oil, thyme oil, pimento berries oil, rose oil, anise oil, balsam oil, bergamot oil, rosewood oil, cedar oil, chamomile oil, sage oil, clary sage oil, clove oil, cypress oil, eucalyptus oil, fennel oil, sea fennel oil, frankincense oil, geranium oil, ginger oil, grapefruit oil, jasmine oil, juniper oil, lavender oil, lemon oil, lemongrass oil, lime oil, mandarin oil, marjoram oil, myrrh oil, neroli oil, orange oil, patchouli oil, pepper oil, black pepper oil, petitgrain oil, pine oil, rose otto oil, rosemary oil, sandalwood oil, spearmint oil, spikenard oil, vetiver oil, wintergreen oil, or ylang ylang. Other essential oils known to those of skill in the art are also contemplated as being useful within the context of the present invention.

### Structuring Agents

The compositions of the present invention can include a structuring agent. Structuring agent, in certain aspects, assist in providing rheological characteristics to the composition to contribute to the composition's stability. In other aspects, structuring agents can also function as an emulsifier or surfactant. Non-limiting examples of structuring agents include stearic acid, palmitic acid, stearyl alcohol, cetyl alcohol, behenyl alcohol, stearic acid, palmitic acid, the polyethylene glycol ether of stearyl alcohol having an average of about 1 to about 21 ethylene oxide units, the polyethylene glycol ether of cetyl alcohol having an average of about 1 to about 5 ethylene oxide units, and mixtures thereof.

### Antioxidants

The composition may further comprise antioxidants. Non-limiting examples of antioxidants that can be used with the compositions of the present invention include acetyl cysteine, ascorbic acid polypeptide, ascorbyl dipalmitate, ascorbyl methylsilanol pectinate, ascorbyl palmitate, ascorbyl stearate, BHA, BHT, t-butyl hydroquinone, cysteine, cysteine HCI, diamylhydroquinone, di-t-butylhydroquinone, dicetyl thiodipropionate, dioleyl tocopheryl methylsilanol, disodium ascorbyl sulfate, distearyl thiodipropionate, ditridecyl thiodipropionate, dodecyl gallate, erythorbic acid, esters of ascorbic acid, ethyl ferulate, ferulic acid, gallic acid esters, hydroquinone, isooctyl thioglycolate, kojic acid, magnesium ascorbate, magnesium ascorbyl phosphate, methylsilanol ascorbate, natural botanical anti-oxidants such as green tea or grape seed extracts, nordihydroguaiaretic acid, octyl gallate, phenylthioglycolic acid, potassium ascorbyl tocopheryl phosphate, potassium sulfite, propyl gallate, quinones, rosmarinic acid, sodium ascorbate, sodium bisulfite, sodium erythorbate, sodium metabisulfite, sodium sulfite, superoxide dismutase, sodium thioglycolate, sorbityl furfural, thiodiglycol, thiodiglycolamide, thiodiglycolic acid, thioglycolic acid, thiolactic acid, thiosalicylic acid, tocophereth-5, tocophereth-10, tocophereth-12, tocophereth-18, tocophereth-50, tocopherol, tocophersolan, tocopheryl acetate, tocopheryl linoleate, tocopheryl nicotinate, tocopheryl succinate, and tris(nonylphenyl)phosphite.

### Chelators

The composition of the present invention may also comprise a safe and effective amount of a chelator or chelating agent. As used herein, "chelator" or "chelating agent" means an active agent capable of removing a metal ion from a system by forming a complex so that the metal ion cannot readily participate in or catalyze chemical reactions. The inclusion of a chelating agent is especially useful for providing protection against UV radiation that can contribute to excessive scaling or skin texture changes and against other environmental agents, which can cause skin damage.

### Anti-Cellulite Agents

The composition of the present invention may also comprise a safe and effective amount of an anti-cellulite agent. Suitable agents may include, but are not limited to, xanthine compounds (e.g., caffeine, theophylline, theobromine, and aminophylline).

### Skin Lightening Aoents

The composition of the present invention may comprise a skin lightening agent. Suitable skin lightening agents include those known in the art, including kojic acid, arbutin, ascorbic acid and derivatives thereof, e.g., magnesium ascorbyl phosphate or sodium ascorbyl phosphate or other salts of ascorbyl phosphate.

### Vitamin Compounds

The composition may comprise vitamin compounds, precursors, and derivatives thereof. These vitamin compounds may be in either natural or synthetic form. Suitable vitamin compounds include, but are not limited to, Vitamin A (e.g., beta carotene, retinoic acid, retinol, retinoids, retinyl palmitate, retinyl proprionate, etc.), Vitamin B (e.g., niacin, niacinamide, riboflavin, pantothenic acid, etc.), Vitamin C (e.g., ascorbic acid, etc.), Vitamin D (e.g., ergosterol, ergocalciferol, cholecalciferol, etc.), Vitamin E (e.g., tocopherol acetate, etc.), and Vitamin K (e.g., phytonadione, menadione, phthiocol, etc.) compounds.

### Moisturizing Agents

The composition may comprise a moisturizing agent. Non-limiting examples of moisturizing agents that can be used with the compositions of the present invention include amino acids, chondroitin sulfate, diglycerin, erythritol, fructose, glucose, glycerin, glycerol polymers, glycol, 1,2,6-hexanetriol, honey, hyaluronic acid, hydrogenated honey, hydrogenated starch hydrolysate, inositol, lactitol, maltitol, maltose, mannitol, natural moisturizing factor, PEG-15 butanediol, polyglyceryl sorbitol, salts of pyrollidone carboxylic acid, potassium PCA, propylene glycol, sodium glucuronate, sodium PCA, sorbitol, sucrose, trehalose, urea, and xylitol. Other examples include acetylated lanolin, acetylated lanolin alcohol, alanine, algae extract, aloe barbadensis, aloe-barbadensis extract, aloe barbadensis gel, althea officinalis extract, apricot (prunus armeniaca) kernel oil, arginine, arginine aspartate, arnica montana extract, aspartic acid, avocado (persea gratissima) oil, barrier sphingolipids, butyl alcohol, beeswax, behenyl alcohol, beta-sitosterol, birch (betula alba) bark extract, borage (borago officinalis) extract, butcherbroom (ruscus aculeatus) extract, butylene glycol, calendula officinalis extract, calendula officinalis oil, candelilla (euphorbia cerifera) wax, canola oil, caprylic/capric triglyceride, cardamon (elettaria cardamomum) oil, carnauba (copernicia cerifera) wax, carrot (daucus carota sativa) oil, castor (ricinus communis) oil, ceramides, ceresin, ceteareth-5, ceteareth-12, ceteareth-20, cetearyl octanoate, ceteth-20, ceteth-24, cetyl acetate, cetyl octanoate, cetyl palmitate, chamomile (anthemis nobilis) oil, cholesterol, cholesterol esters, cholesteryl hydroxystearate, citric acid, clary (salvia sclarea) oil, cocoa (theobroma cacao) butter, coco-caprylate/caprate, coconut (cocos nucifera) oil, collagen, collagen amino acids, corn (zea mays) oil, fatty acids, decyl oleate, dimethicone copolyol, dimethiconol, dioctyl adipate, dioctyl succinate, dipentaerythrityl hexacaprylate/hexacaprate, DNA, erythritol, ethoxydiglycol, ethyl linoleate, eucalyptus globulus oil, evening primrose (oenothera biennis) oil, fatty acids, geranium maculatum oil, glucosamine, glucose glutamate, glutamic acid, glycereth-26, glycerin, glycerol, glyceryl distearate, glyceryl hydroxystearate, glyceryl laurate, glyceryl linoleate, glyceryl myristate, glyceryl oleate, glyceryl stearate, glyceryl stearate SE, glycine, glycol stearate, glycol stearate SE, glycosaminoglycans, grape (vitis vinifera) seed oil, hazel (corylus americana) nut oil, hazel (corylus avellana) nut oil, hexylene glycol, hyaluronic acid, hybrid safflower (carthamus tinctorius) oil, hydrogenated castor oil, hydrogenated coco-glycerides, hydrogenated coconut oil, hydrogenated lanolin, hydrogenated lecithin, hydrogenated palm glyceride, hydrogenated palm kernel oil, hydrogenated soybean oil, hydrogenated tallow glyceride, hydrogenated vegetable oil, hydrolyzed collagen, hydrolyzed elastin, hydrolyzed glycosaminoglycans, hydrolyzed keratin, hydrolyzed soy protein, hydroxylated lanolin, hydroxyproline, isocetyl stearate, isocetyl stearoyl stearate, isodecyl oleate, isopropyl isostearate, isopropyl lanolate, isopropyl myristate, isopropyl palmitate, isopropyl stearate, isostearamide DEA, isostearic acid, isostearyl lactate, isostearyl neopentanoate, jasmine (jasminum officinale) oil, jojoba (buxus chinensis) oil, kelp, kukui (aleurites moluccana) nut oil, lactamide MEA, laneth-16, laneth-10 acetate, lanolin, lanolin acid, lanolin alcohol, lanolin oil, lanolin wax, lavender (lavandula angustifolia) oil, lecithin, lemon (citrus medica limonum) oil, linoleic acid, linolenic acid, macadamia ternifolia nut oil, maltitol, matricaria (chamomilla recutita) oil, methyl glucose sesquistearate, methylsilanol PCA, mineral oil, mink oil, mortierella oil, myristyl lactate, myristyl myristate, myristyl propionate, neopentyl glycol dicaprylate/dicaprate, octyldodecanol, octyldodecyl myristate, octyldodecyl stearoyl stearate, octyl hydroxystearate, octyl palmitate, octyl salicylate, octyl stearate, oleic acid, olive (olea europaea) oil, orange (citrus aurantium dulcis) oil, palm (elaeis guineensis) oil, palmitic acid, pantethine, panthenol, panthenyl ethyl ether, paraffin, PCA, peach (prunus persica) kernel oil, peanut (arachis hypogaea) oil, PEG-8 C12-18 ester, PEG-15 cocamine, PEG-150 distearate, PEG-60 glyceryl isostearate, PEG-5 glyceryl stearate, PEG-30 glyceryl stearate, PEG-7 hydrogenated castor oil, PEG-40 hydrogenated castor oil, PEG-60 hydrogenated castor oil, PEG-20 methyl glucose sesquistearate, PEG40 sorbitan peroleate, PEG-5 soy sterol, PEG-10 soy sterol, PEG-2 stearate, PEG-8 stearate, PEG-20 stearate, PEG-32 stearate, PEG40 stearate, PEG-50 stearate, PEG-100 stearate, PEG-150 stearate, pentadecalactone, peppermint (mentha piperita) oil, petrolatum, phospholipids, polyamino sugar condensate, polyglyceryl-3 diisostearate, polyquaternium-24, polysorbate 20, polysorbate 40, polysorbate 60, polysorbate 80, polysorbate 85, potassium myristate, potassium palmitate, propylene glycol, propylene glycol dicaprylate/dicaprate, propylene glycol dioctanoate, propylene glycol dipelargonate, propylene glycol laurate, propylene glycol stearate, propylene glycol stearate SE, PVP, pyridoxine dipalmitate, retinol, retinol palmitate, rice (oryza sativa) bran oil, RNA, rosemary (rosmarinus officinalis) oil, rose oil, safflower (carthamus tinctorius) oil, sage (salvia officinalis) oil, sandalwood (santalum album) oil, serine, serum protein, sesame (sesamum indicum) oil, shea butter (butyrospermum parkii), silk powder, sodium chondroitin sulfate, sodium hyaluronate, sodium lactate, sodium palmitate, sodium PCA, sodium polyglutamate, soluble collagen, sorbitan laurate, sorbitan oleate, sorbitan palmitate, sorbitan sesquioleate, sorbitan stearate, sorbitol, soybean (glycine soja) oil, sphingolipids, squalane, squalene, stearamide MEA-stearate, stearic acid, stearoxy dimethicone, stearoxytrimethylsilane, stearyl alcohol, stearyl glycyrrhetinate, stearyl heptanoate, stearyl stearate, sunflower (helianthus annuus) seed oil, sweet almond (prunus amygdalus dulcis) oil, synthetic beeswax, tocopherol, tocopheryl acetate, tocopheryl linoleate, tribehenin, tridecyl neopentanoate, tridecyl stearate, triethanolamine, tristearin, urea, vegetable oil, water, waxes, wheat (triticum vulgare) germ oil, and ylang ylang (cananga odorata) oil.

### Skin Treating Agents

The composition of the present invention may contain one or more skin treating agents. Suitable skin treating agents include those effective for preventing, retarding, arresting, and/or reversing skin wrinkles. Examples of suitable skin treating agents include, but are not limited to, alpha-hydroxy acids such as lactic acid and glycolic acid and beta-hydroxy acids such as salicylic acid.

### Anti-Acne Actives

Examples of useful anti-acne actives for the composition of the present invention include, but are not limited to, the keratolytics such as salicylic acid (o-hydroxybenzoic acid), derivatives of salicylic acid such as 5-octanoyl salicylic acid, and resorcinol; retinoids such as retinoic acid and its derivatives (e.g., cis and trans); sulfur-containing D and L amino acids and their derivatives and salts, particularly their N-acetyl derivatives, a preferred example of which is N-acetyl-L-cysteine; lipoic acid; antibiotics and antimicrobials such as benzoyl peroxide, octopirox, tetracycline, 2,4,4'-trichloro-2'-hydroxy diphenyl ether, 3,4,4'-trichlorobanilide, azelaic acid and its derivatives, phenoxyethanol, phenoxypropanol, phenoxyisopropanol, ethyl acetate, clindamycin and meclocycline; sebostats such as flavonoids; and bile salts such as scymnol sulfate and its derivatives, deoxycholate, and cholate.

### Anti-Wrinkle and Anti-Skin Atrophy Actives

Examples of anti-wrinkle and anti-skin atrophy actives useful for the composition of the present invention include, but are not limited to, retinoic acid and its derivatives (e.g., cis and trans); retinol; retinyl esters; niacinamide, salicylic acid and derivatives thereof; sulfur-containing D and L amino acids and their derivatives and salts, particularly the N-acetyl derivatives, a preferred example of which is N-acetyl-L-cysteine; thiols, e.g., ethane thiol; hydroxy acids, phytic acid, lipoic acid; lysophosphatidic acid, and skin peel agents (e.g., phenol and the like).

### Non-Steroidal Anti-inflammatory Actives (NSAIDS)

Examples of NSAIDS useful for the composition of the present invention include, but are not limited to, the following categories: propionic acid derivatives; acetic acid derivatives; fenamic acid derivatives; biphenylcarboxylic acid derivatives; and oxicams.

### Topical Anesthetics

Examples of topical anesthetic drugs useful for the composition of the present invention include, but are not limited to, benzocaine, lidocaine, bupivacaine, chlorprocaine, dibucaine, etidocaine, mepivacaine, tetracaine, dyclonine, hexylcaine, procaine, cocaine, ketamine, pramoxine, phenol, and pharmaceutically acceptable salts thereof.

### Antimicrobial and Antifungal Actives

Examples of antimicrobial and antifungal actives useful for the composition of the present invention include, but are not limited to, .beta.-lactam drugs, quinolone drugs, ciprofloxacin, norfloxacin, tetracycline, erythromycin, amikacin, 2,4,4'-trichloro-2'-hydroxy diphenyl ether, 3,4,4'-trichlorocarbanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearatc, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amanfadine sulfate, octopirox, parachlorometa xylenol, nystatin, tolnaftate, zinc pyrithione, Climbazole and clotrimazole.

### Enzymes

The composition of the present invention may optionally include one or more enzymes. Preferably, such enzymes are dermatologically acceptable. Suitable enzymes include, but are not limited to, keratinase, protease, amylase, subtilisin, etc.

### Sunscreen Actives

UV absorption agents that can be used in the composition of the present invention include chemical and physical sunblocks. Non-limiting examples of chemical sunblocks that can be used include para-aminobenzoic acid (PABA), PABA esters (glyceryl PABA, amyldimethyl PABA and octyldimethyl PABA), butyl PABA, ethyl PABA, ethyl dihydroxypropyl PABA, benzophenones (oxybenzone, sulisobenzone, benzophenone, and benzophenone-1 through 12), cinnamates (octyl methoxycinnamate, isoamyl p-methoxycinnamate, octylmethoxy cinnamate, cinoxate, diisopropyl methyl cinnamate, DEA-methoxycinnamate, ethyl diisopropylcinnamate, glyceryl octanoate dimethoxycinnamate and ethyl methoxycinnamate), cinnamate esters, salicylates (homomethyl salicylate, benzyl salicylate, glycol salicylate, isopropylbenzyl salicylate, etc.), anthranilates, ethyl urocanate, homosalate, octisalate, dibenzoylmethane derivatives (e.g., avobenzone), octocrylene, octyl triazone, digalloy trioleate, glyceryl aminobenzoate, lawsone with dihydroxyacetone, ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutyiphenol, and bis-ethylhexyloxyphenol methoxyphenyltriazine, 4-methylbenzylidenecamphor, and isopentyl 4-methoxycinnamate. Non-limiting examples of physical sunblocks include, kaolin, talc, petrolatum and metal oxides (e.g., titanium dioxide and zinc oxide).

### Thickening Agents

Thickening agents, including thickener or gelling agents, include substances which that can increase the viscosity of a composition. Thickeners includes those that can increase the viscosity of a composition without substantially modifying the efficacy of the active ingredient within the composition. Thickeners can also increase the stability of the compositions of the present invention. In certain aspects of the present invention, thickeners include hydrogenated polyisobutene or trihydroxystearin, or a mixture of both.

Non-limiting examples of additional thickening agents that can be used in the context of the present invention include carboxylic acid polymers, crosslinked polyacrylate polymers, polyacrylamide polymers, polysaccharides, and gums. Examples of carboxylic acid polymers include crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. Examples of commercially available carboxylic acid polymers include carbomers, which are homopolymers of acrylic acid crosslinked with allyl ethers of sucrose or pentaerytritol.

Non-limiting examples of polysaccharides include cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Another example is an alkyl substituted cellulose where the hydroxy groups of the cellulose polymer is hydroxyalkylated (preferably hydroxy ethylated or hydroxypropylated) to form a hydroxyalkylated cellulose which is then further modified with a C.sub.**1**0-C.sub.30 straight chain or branched chain alkyl group through an ether linkage. Typically these polymers are ethers of C.sub.**1**0-C.sub.30 straight or branched chain alcohols with hydroxyalkylcelluloses. Other useful polysaccharides include scleroglucans comprising a linear chain of (1-3) linked glucose units with a (1-6) linked glucose every three unit.

Non-limiting examples of gums that can be used with the present invention include acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof.

### Cationic Surfactants

The composition may further comprise a cationic surfactant, such as a quaternary ammonium salt cationic surfactant. Nonlimiting examples of quaternary ammonium salt cationic surfactants include those selected from the group consisting of cetyl ammonium chloride, cetyl ammonium bromide, lauryl ammonium chloride, lauryl ammonium bromide, stearyl ammonium chloride, stearyl ammonium bromide, cetyl dimethyl ammonium chloride, cetyl dimethyl ammonium bromide, lauryl dimethyl ammonium chloride, lauryl dimethyl ammonium bromide, stearyl dimethyl ammonium chloride, stearyl dimethyl ammonium bromide, cetyl trimethyl ammonium chloride, cetyl trimethyl ammonium bromide, lauryl trimethyl ammonium chloride, lauryl trimethyl ammonium bromide, stearyl trimethyl ammonium chloride, stearyl trimethyl ammonium bromide, lauryl dimethyl ammonium chloride, stearyl dimethyl cetyl ditallow dimethyl ammonium chloride, dicetyl ammonium chloride, dicetyl ammonium bromide, dilauryl ammonium chloride, dilauryl ammonium bromide, distearyl ammonium chloride, distearyl ammonium bromide, dicetyl methyl ammonium chloride, dicetyl methyl ammonium bromide, dilauryl methyl ammonium chloride, dilauryl methyl ammonium bromide, distearyl methyl ammonium chloride, distearyl dimethyl ammonium chloride, distearyl methyl ammonium bromide, and mixtures thereof. Suitable cationic quaternary ammonium salt also include ditallow dimethyl ammonium chloride, ditallow dimethyl ammonium methyl sulfate, di(hydrogenated tallow) dimethyl ammonium chloride, di(hydrogenated tallow) dimethyl ammonium acetate, ditallow dipropyl ammonium phosphate, ditallow dimethyl ammonium nitrate, di(coconutalkyl)dimethyl ammonium chloride, di(coconutalkyl)dimethyl ammonium bromide, tallow ammonium chloride, coconut ammonium chloride, stearamidopropyl ethyidimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, and mixtures thereof.

The emulsion composition of the present invention is typically an oil-in-polyol emulsion which comprises a high content of oil. One challenge associated with such compositions is that it is difficult to maintain the stability of the composition and to prevent phase separation of different components. It has been found that the emulsion composition according to the present invention can have excellent stability such that the components would form homogeneous mixture and no phase separation occurs. In addition, the stability of the composition can be achieved by addition of a relatively low amount of amphoteric surfactant alone and it does not necessarily require addition of other surfactants, such as ethoxylated nonionic surfactants which are commonly used as emulsifier for emulsions. This is particularly advantageous considering the potential hazardous impacts to the environment which may be caused by ethoxylated surfactants.

The emulsion composition of the present invention can be provided in the form of liquid, paste, cream, gel or the like. The composition can be used for personal care, pharmaceutical and cosmetic products, such as cleaning foams and gels, body lotions and milks, makeup remover oils, massage oils, therapeutical oils or creams, hair oils and anti UV gels. The composition may also be used for other external skin compositions, such as moisturizing, anti-aging, whitening, pack, foundation, lip cream formulations.

The emulsion composition may be prepared by mixing the above-described components using conventional blending methods. Suitable blending methods can be readily determined in accordance with blending tests for the individual components, which are commonly conducted by those skilled in the art. The emulsion composition is generally combined by mixing the individual components at a temperature higher than their melting points to melt them and cooling the melt to about room temperature with stirring. Typically, the method include a step of preparing a mixture of the polyol and the amphoteric surfactant under heating (about 40 °C to 90 °C), then the resulting mixture can be mixed with the oil phase to obtain the emulsion composition of the present invention. Water and other optional components can also be added to the emulsion composition by using blending techniques generally known in the art.

The examples provided here further describe and demonstrate embodiments of the present invention. The examples are given solely for the purpose of illustration and are not to be construed as limitation of the present invention.

### Examples

### Materials:

Cetyl betaine from the Solvay company;
Erucamidopropyl hydroxysultaine from the Solvay company.

Sodium stearoampho acetate from the Solvay company;
26# white oil from the Sasol company;
Caprylic/capric triglyceride from the Croda company;
Dicaprylyl carbonate from the BASF company;
Isopropyl myristate from the Evonik company;
Polysorbate 20 from the Solvay company;
Ceteareth-25 from the Solvay company;
PEG-40 hydrogenated Castor Oil from the Solvay company.

### Example 1

Emulsion composition samples were prepared according to the formulations in Table 1 below (S means Sample and CS means Comparative Sample):

**Table 1**

| **Samples** | | **S1** | **S2** | **S3** | **S4** | **CS1** |
|---|---|---|---|---|---|---|
| **Components (wt%)** | | | | | | |
| **A** | **Cetyl betaine (30% Active)** | 2.5 | 2.5 | - | - | 2.5 |
| | **Erucamidopropyl hydroxysultaine (45% Active**) | - | - | 2.33 | - | - |
| | **Sodium stearoampho acetate (20% Active)** | - | - | - | 2.5 | - |
| | **Glycerol** | 10.585 | 10.43 | 12.25 | 10.585 | 25.585 |
| | **Liquid sorbitol (70 wt% aqueous solution)** | 10 | 10.26 | 12.2 | 10 | 11.915 |
| **B** | **26# white oil** | 50 | 50 | 47 | 50 | 25 |
| | **Caprylic/capric triglyceride** | - | 10 | - | | |
| | **Dicaprylyl carbonate** | - | 15 | - | | |
| | **Isopropyl myristate** | 25 | - | 23 | 25 | 25 |
| **D.I. Water** | | 1.915 | 1.81 | 3.22 | 1.915 | 10 |
| **Total** | | 100 | 100 | 100 | 100 | 100 |

For the preparation, components in Phase A were mixed together and stirred until a homogenous mixture formed. Then the Phase A mixture was heated to 70-75 °C. Components in Phase B were mixed and the resulting mixture was added drop-wise into Phase A with stirring. The mixture of Phase A and Phase B was then cooled to a temperature below 45 °C. Subsequently D.I. water was added to the mixture.

The emulsion compositions samples were kept at the ambient temperature for 48 hrs. Then the physical appearance of the samples was observed. Results are indicated in Table 2 below:

**Table 2**

| **S1** | **S2** | **S3** | **S4** | **CS1** |
|---|---|---|---|---|
| Transparent gel, homogenous | Transparent gel, homogenous | Transparent gel, homogenous | Translucent gel, homogenous | Separation of **oil** phase and polyol phase |

Results showed that the emulsion compositions according to the present invention showed excellent stability and no phase separation were observed after 48 hrs.

### Example 2

Emulsion composition samples were prepared according to the formulations in Table 3 below and according to the process as described in Example 1:

**Table 3**

| **Samples** | | **S5** | **CS2** |
|---|---|---|---|
| **Components (wt%)** | | | |
| **A** | **Cetyl betaine (30% Active)** | 2 | 1 |
| | **Polysorbate 20** | - | 1 |
| | **Glycerol** | 23.6 | 23.6 |
| **B** | **26# white oil** | 74.4 | 74.4 |
| **Total** | | 100 | 100 |

The emulsion compositions samples were kept at the ambient temperature for 48 hrs. Then the physical appearance of the samples was observed. Results are indicated in Table 4 below:

**Table 4**

| **S5** | **CS2** |
|---|---|
| Transparent gel, homogenous | Separation of oil phase and polyol phase |

Results showed that the emulsion composition according to the present invention exhibited excellent stability while the emulsion composition comprising both an amphoteric surfactant and a nonionic surfactant (i.e., polysorbate 20) exhibited poor stability and undergone phase separation after 48 hrs.

### Example 3

In another set of experiments, emulsion composition samples were prepared according to the formulations in Table 5 below. The samples were prepared and treated according to the procedures as in Example 1. The physical appearance of the treated samples was shown in Table 6.

**Table 5**

| **Samples** | | **S6** | **S7** | **S8** | **CS3** |
|---|---|---|---|---|---|
| **A** | **Cetyl betaine (powder)** | 5 | - | **1** | 1 |
| | **Erucamidopropyl hydroxysultaine (Powder)** | 2.5 | 5 | - | - |
| | **Sodium stearoampho acetate (powder)** | - | 2.5 | 1 | - |
| | **Glycerol** | 15 | 5 | 7 | 6 |
| | **Liquid sorbitol (70 wt% aqueous solution)** | 2.5 | 5.5 | - | - |
| **B** | **26# white oil** | 30 | 50 | 50 | 70 |
| | **Caprylic/capric triglyceride** | 20 | - | 20 | 22 |
| | **Dicaprylyl carbonate** | 10 | 22 | 10 | - |
| | **Isopropyl myristate** | 13 | 9.5 | 10 | - |
| **D.I. Water** | | 2 | 0.5 | 1 | 2 |
| Total | | 100 | 100 | 100 | 100 |

**Table 6**

| **S6** | **S7** | **S8** | **CS3** |
|---|---|---|---|
| Translucent gel, homogenous | Translucent gel, homogenous | Translucent gel, homogenous | Homogenous emulsion was not achievable |

### Example 4

In another set of experiments, emulsion composition samples were prepared according to the formulation in Table 7 below. The samples were prepared and treated according to the procedures as in Example 1. The physical appearance of the treated samples was shown in Table 8.

**Table 7**

| **Samples** | | **S9** | **CS4** | **CS5** | **CS6** |
|---|---|---|---|---|---|
| **Components (wt%)** | | | | | |
| **A** | **Cetyl betaine (Powder)** | 0.6 | 1 | - | - |
| | **Erucamidopropyl hydroxysultaine (Powder)** | - | - | 1 | - |
| | **Sodium stearoampho acetate (powder)** | - | - | - | 1 |
| | **Polysorbate 20** | - | - | - | 0.8 |
| | **Ceteareth-25** | - | 0.8 | - | - |
| | **PEG-40 hydrogenated Castor Oil** | - | - | 0.8 | - |
| | **Glycerol** | 23.6 | 20.5 | 20.5 | 20.5 |
| **B** | **26# white oil** | 74.4 | 75 | 75 | 75 |
| **D.I. Water** | | 1.4 | 2.7 | 2.7 | 2.7 |
| **Total** | | 100 | 100 | 100 | 100 |

**Table 8**

| **S9** | **CS4** | **CS5** | **CS6** |
|---|---|---|---|
| Transparent gel, homogenous | Separation of oil phase and polyol phase | Separation of oil phase and polyol phase | Separation of oil phase and polyol phase |

Results showed that the composition comprising the amphoteric surfactant according to the present invention exhibited transparent and homogenous emulsion. In contrast, the compositions comprising nonionic surfactants (polysorbate 20, ceteareth-25 or PEG-40 hydrogenated Castor Oil) showed phase separation after 48 hrs.

## Claims

1. An emulsion composition comprising :
(a) from 65 wt% to 90 wt% of an oil phase;
(b) from 5 wt% to 30 wt% of a polyol;
(c) from 0.01 wt% to 7.5 wt% of an amphoteric surfactant;
(d) from 0 wt% to 0.5 wt% of a nonionic surfactant;
percent by weight is based on the total weight of the composition.

2. The emulsion composition according to claim 1, wherein the composition comprises :
(a) from 70 wt% to 90 wt% of an oil phase;
(b) from 5 wt% to 25 wt% of a polyol;
(c) from 0.01 to 7.5 wt% of an amphoteric surfactant;
(d) from 0 wt% to 0.5 wt% of a nonionic surfactant;
percent by weight is based on the total weight of the composition.

3. The emulsion composition according to claim 1 or 2, wherein the composition comprises :
(a) from 70 wt% to 90 wt% of an oil phase;
(b) from 5 wt% to 25 wt% of a polyol;
(c) from 0.01 to 5 wt% of an amphoteric surfactant;
(d) from 0 wt% to 0.5 wt% of a nonionic surfactant;
percent by weight is based on the total weight of the composition.

4. The emulsion composition according to any one of claims 1 to 3,
wherein the emulsion composition comprises less than 0.1 wt%, more preferably less than 0.01 wt%, for instance 0 wt% of nonionic surfactant based on the total weight of the composition.

5. The emulsion composition according to any one of claims 1 to 4,
wherein the emulsion composition comprises from 0.01 wt% to 5 wt% of water based on the total weight of the composition.

6. The emulsion composition according to any one of claims 1 to 5,
wherein the amphoteric surfactant is chosen from the group consisting of: betaine, amine oxide, amphoglycinate and amphopropionate.

7. The emulsion composition according to any one of claims 1 to 6,
wherein the amphoteric surfactant is a compound having the general formula of:
**R₁ R₂ R₃ N⁺** (CH₂)_{y} **C(O)O⁻** **(I)**
or
**(R₁)(R₂C(O)NH(CH₂)ₓ)(R₃)N⁺(CH₂)_{y} C(O)O⁻** **(II)**
wherein R₁ is a C₁-C₅ alkyl or alkenyl group which is optionally hydroxylated;
R₂ is a C₁₀-C₂₂-alkyl or C₁₀-C₂₂-alkenyl group;
R₃ is independently selected from a C₁-C₅ alkyl or alkenyl group, C₁₀-C₂₂-alkyl or C₁₀-C₂₂-alkenyl group;
x is an integer of 2-4, y is an integer of 1-4.

8. The emulsion composition according to any one of claims 1 to 7,
wherein the amphoteric surfactant is a compound having the general formula of:
**R₇-N⁺(CH₃)₂-CH₂-COO⁻** **(IX)**
wherein R₇ is a C₁₀-C₂₂-alkyl or C₁₀-C₂₂-alkenyl group.

9. The emulsion composition according to claim 8, wherein R₇ is a C₁₆-C₂₂-alkyl or C₁₆-C₂₂-alkenyl group.

10. The emulsion composition according to any one of claims 1 to 6,
wherein the amphoteric surfactant is a compound having the general formula of:
**R₁ R₂ R₃ N⁺R₄-SO₃** **(III)**
or
**(R₁)(R₂C(O)NH(CH₂)ₓ)(R₃)N⁺R₄-SO₃⁻** **(IV)**
wherein R₁ is a C₁-C₅ alkyl or alkenyl group which is optionally hydroxylated;
R₂ is a C₁₀-C₂₂-alkyl or C₁₀-C₂₂-alkenyl group;
R₃ is independently selected from a C₁-C₅ alkyl or alkenyl group, C₁₀-C₂₂-alkyl or C₁₀-C₂₂-alkenyl group;
R₄ is a C₁₋C₄ alkyl group, linear or branched, optionally hydroxylated;
x is an integer of 2-4.

11. The emulsion composition according to any one of claims 1 to 6,
wherein the amphoteric surfactant is a compound having the general formula of:
**R₁ R₂ R₃ N⁺ --O⁻** **(V)**
or
**(R₁)(R₂C(O)NH(CH₂)ₓ)(R₃)N⁺--O⁻** **(VI)**
wherein R₁ is a C₁-C₅ alkyl or alkenyl group which is optionally hydroxylated;
R₂ is a C₁₀-C₂₂-alkyl or C₁₀-C₂₂-alkenyl group;
R₃ is independently selected from a C₁-C₅ alkyl or alkenyl group, C₁₀-C₂₂-alkyl or C₁₀-C₂₂-alkenyl group;
x is an integer of 2-4.

12. The emulsion composition according to any one of claims 1 to 6,
wherein the amphoteric surfactant is a compound having the general formula of:
**R₂ N(R₅)(CH₂)_{z} C(O)O⁻ Y⁺** **(VII)**
or
**R₂ C(O)N(R₅)(CH₂)x N(R₆)(CH₂), C(O)O⁻ Y⁺** **(VIII)**
wherein R₂ is a C₁₀-C₂₂-alkyl or C₁₀-C₂₂-alkenyl group;
R₅ is hydrogen or a C₁-C₅ group which is optionally hydroxylated;
R₆ is a C₁-C₅ group which is optionally hydroxylated or a (CH₂)_{z} C(O)O⁻ group;
z is an integer of 1-4;
x is an integer of 2-4;
Y**⁺** is a cation.

13. The emulsion composition according to any one of claims 1 to 12, wherein the polyol is a compound having the formula :
**R₈-C(-R₉)(-OH)-[A-C(-OH)(R₁₀)]ₙ-R₁₁** **(X)**
wherein
R₈, R₉, R₁₀ and R₁₁ are each independently selected from the group comprising: hydrogen, C₁-C₆ hydrocarbyl, C₁-C₆ cycloalkyl and C₁-C₆ heterocyclic moiety; said hydrocarbyl moiety is saturated or unsaturated, branched or linear; each of said hydrocarbyl, cycloalkyl and heterocyclic moiety is optionally substituted with one or several substituents;
A is a covalent bond or a linking moiety;
n is an integer of 1 to 10.

14. The emulsion composition according to any one of claims 1 to 13, wherein the polyol is selected from propylene glycol, isoprene glycol, 1,3-butanediol, dipropylene glycol, glycerol, diglycerol, triglycerol, polyglycerol, trimethylolpropane, erythritol, pentaerythritol, sorbitan, sorbitol, glucose, maltitol, saccharose, trehalose, polyethylene glycol and a mixture thereof.

15. The emulsion composition according to any one of claims 1 to 14, wherein the emulsion composition further comprises a perfume.

## Patentansprüche

1. Emulsionszusammensetzung, umfassend:
(a) 65 Gew.-% bis 90 Gew.-% einer Ölphase;
(b) 5 Gew.-% bis 30 Gew.-% eines Polyols;
(c) 0,01 Gew.-% bis 7,5 Gew.-% eines amphoteren Tensids;
(d) 0 Gew.-% bis 0,5 Gew.-% eines nichtionischen Tensids;
wobei sich die Gewichtsprozentangaben auf das Gesamtgewicht der Zusammensetzung beziehen.

2. Emulsionszusammensetzung nach Anspruch 1, wobei die Zusammensetzung Folgendes umfasst:
(a) 70 Gew.-% bis 90 Gew.-% einer Ölphase;
(b) 5 Gew.-% bis 25 Gew.-% eines Polyols;
(c) 0,01 Gew.-% bis 7,5 Gew.-% eines amphoteren Tensids;
(d) 0 Gew.-% bis 0,5 Gew.-% eines nichtionischen Tensids;
wobei sich die Gewichtsprozentangaben auf das Gesamtgewicht der Zusammensetzung beziehen.

3. Emulsionszusammensetzung nach Anspruch 1 oder 2, wobei die Zusammensetzung Folgendes umfasst:
(a) 70 Gew.-% bis 90 Gew.-% einer Ölphase;
(b) 5 Gew.-% bis 25 Gew.-% eines Polyols;
(c) 0,01 Gew.-% bis 5 Gew.-% eines amphoteren Tensids;
(d) 0 Gew.-% bis 0,5 Gew.-% eines nichtionischen Tensids;
wobei sich die Gewichtsprozentangaben auf das Gesamtgewicht der Zusammensetzung beziehen.

4. Emulsionszusammensetzung nach einem der Ansprüche 1 bis 3, wobei die Emulsionszusammensetzung weniger als 0,1 Gew.-%, weiter bevorzugt weniger als 0,01 Gew.-%, beispielsweise 0 Gew.-%, nichtionisches Tensid, bezogen auf das Gesamtgewicht der Zusammensetzung, umfasst.

5. Emulsionszusammensetzung nach einem der Ansprüche 1 bis 4, wobei die Emulsionszusammensetzung 0,01 Gew.-% bis 5 Gew.-% Wasser, bezogen auf das Gesamtgewicht der Zusammensetzung, umfasst.

6. Emulsionszusammensetzung nach einem der Ansprüche 1 bis 5, wobei das amphotere Tensid aus der Gruppe bestehend aus Betain, Aminoxid, Amphoglycinat und Amphopropionat ausgewählt ist.

7. Emulsionszusammensetzung nach einem der Ansprüche 1 bis 6, wobei es sich bei dem amphoteren Tensid um eine Verbindung mit der folgenden allgemeinen Formel handelt:
**R₁ R₂ R₃ N⁺ (CH₂)_{y} C(O)O⁻** **(I)**
oder
**(R₁) (R₂C(O)NH(CH₂)ₓ) (R₃)N⁺(CH₂)_{y} C(O)O⁻** **(II)**
wobei R₁ für eine gegebenenfalls hydroxylierte C₁-C₅-Alkyl- oder -Alkenylgruppe steht;
R₂ für eine C₁₀-C₂₂-Alkyl- oder C₁₀-C₂₂-Alkenylgruppe steht;
R₃ unabhängig aus einer C₁-C₅-Alkyl- oder -Alkenylgruppe oder C₁₀-C₂₂-Alkyl- oder C₁₀-C₂₂-Alkenylgruppe ausgewählt ist;
x für eine ganze Zahl von 2-4 steht und y für eine ganze Zahl von 1-4 steht.

8. Emulsionszusammensetzung nach einem der Ansprüche 1 bis 7, wobei es sich bei dem amphoteren Tensid um eine Verbindung mit der folgenden allgemeinen Formel handelt:
**R₇-N⁺ (CH₃)₂-CH₂-COO⁻** **(IX)**
wobei R₇ für eine C₁₀-C₂₂-Alkyl- oder C₁₀-C₂₂-Alkenylgruppe steht.

9. Emulsionszusammensetzung nach Anspruch 8, wobei R₇ für eine C₁₆-C₂₂-Alkyl- oder C₁₆-C₂₂-Alkenylgruppe steht.

10. Emulsionszusammensetzung nach einem der Ansprüche 1 bis 6, wobei es sich bei dem amphoteren Tensid um eine Verbindung mit der folgenden allgemeinen Formel handelt:
R₁ R₂ R₃ **N⁺R₄-SO₃** **(III)**
oder
**(R₁) (R₂C(O)NH(CH₂)ₓ) (R₃)N⁺ R₄-SO₃⁻** **(IV)**
wobei R₁ für eine gegebenenfalls hydroxylierte C₁-C₅-Alkyl- oder -Alkenylgruppe steht;
R₂ für eine C₁₀-C₂₂-Alkyl- oder C₁₀-C₂₂-Alkenylgruppe steht;
R₃ unabhängig aus einer C₁-C₅-Alkyl- oder -Alkenylgruppe oder C₁₀-C₂₂-Alkyl- oder C₁₀₋C₂₂-Alkenylgruppe ausgewählt ist;
R₄ für eine lineare oder verzweigte, gegebenenfalls hydroxylierte C₁-C₄-Alkylgruppe steht;
x für eine ganze Zahl von 2-4 steht.

11. Emulsionszusammensetzung nach einem der Ansprüche 1 bis 6, wobei es sich bei dem amphoteren Tensid um eine Verbindung mit der folgenden allgemeinen Formel handelt:
**R₁ R₂ R₃ N⁺ --O⁻** **(V)**
oder
**(R₁) (R₂C(O)NH(CH₂)ₓ) (R₃)N⁺--0⁻** **(VI)**
wobei R₁ für eine gegebenenfalls hydroxylierte C₁-C₅-Alkyl- oder -Alkenylgruppe steht;
R₂ für eine C₁₀-C₂₂-Alkyl- oder C₁₀-C₂₂-Alkenylgruppe steht;
R₃ unabhängig aus einer C₁-C₅-Alkyl- oder -Alkenylgruppe oder C₁₀-C₂₂-Alkyl- oder C₁₀-C₂₂-Alkenylgruppe ausgewählt ist;
x für eine ganze Zahl von 2-4 steht.

12. Emulsionszusammensetzung nach einem der Ansprüche 1 bis 6, wobei es sich bei dem amphoteren Tensid um eine Verbindung mit der folgenden allgemeinen Formel handelt:
**R₂ N(R₅) (CH₂)_{z} C(O)O⁻ Y⁺** **(VII**)
oder
**R₂ C(O)N(R₅) (CH₂)ₓ N(R₆) (CH₂)_{z} C(O)O⁻ Y⁺** **(VIII)**
wobei R₂ für eine C₁₀-C₂₂-Alkyl- oder C₁₀-C₂₂-Alkenylgruppe steht;
R₅ für Wasserstoff oder eine gegebenenfalls hydroxylierte C₁-C₅-Gruppe steht;
R₆ für eine gegebenenfalls hydroxylierte C₁-C₅-Gruppe oder eine (CH₂)_{z} C(O)O⁻ Gruppe steht;
z für eine ganze Zahl von 1-4 steht;
x für eine ganze Zahl von 2-4 steht;
Y⁺ für ein Kation steht.

13. Emulsionszusammensetzung nach einem der Ansprüche 1 bis 12, wobei es sich bei dem Polyol um eine Verbindung mit der folgenden Formel handelt:
**R₈-C(-R₉)(-OH)-[A-C(-OH)(R₁₀)]ₙ-R₁₁** **(X)**
wobei
R₈, R₉, R₁₀ und R₁₁ jeweils unabhängig aus der Gruppe umfassend Wasserstoff, eine C₁-C₆-Hydrocarbyl-, C₁-C₆-Cycloalkyl- und C₁-C₆-heterocyclische Gruppierung ausgewählt sind, wobei die Hydrocarbylgruppierung gesättigt oder ungesättigt und verzweigt oder linear ist, wobei die Hydrocarbyl-, Cycloalkyl- und heterocyclische Gruppierung jeweils gegebenenfalls durch einen oder mehrere Substituenten substituiert sind;
A für eine kovalente Bindung oder eine Verknüpfungsgruppierung steht;
n für eine ganze Zahl von 1 bis 10 steht.

14. Emulsionszusammensetzung nach einem der Ansprüche 1 bis 13, wobei das Polyol aus Propylenglykol, Isoprenglykol, 1,3-Butandiol, Dipropylenglykol, Glycerin, Diglycerin, Triglycerin, Polyglycerin, Trimethylolpropan, Erythritol, Pentaerythritol, Sorbitan, Sorbitol, Glucose, Maltitol, Saccharose, Trehalose, Polyethylenglykol und einer Mischung davon ausgewählt ist.

15. Emotionszusammensetzung nach einem der Ansprüche 1 bis 14, wobei die Emulsionszusammensetzung ferner einen Duftstoff umfasst.

## Revendications

1. Composition d'émulsion comprenant :
(a) de 65 % en poids à 90 % en poids d'une phase d'huile ;
(b) de 5 % en poids à 30 % en poids d'un polyol ;
(c) de 0,01 % en poids à 7,5 % en poids d'un tensioactif amphotère ;
(d) de 0 % en poids à 0,5 % en poids d'un tensioactif non ionique ;
les pourcentages en poids étant basés sur le poids total de la composition.

2. Composition d'émulsion selon la revendication 1, la composition comprenant :
(a) de 70 % en poids à 90 % en poids d'une phase d'huile ;
(b) de 5 % en poids à 25 % en poids d'un polyol ;
(c) de 0,01 % en poids à 7,5 % en poids d'un tensioactif amphotère ;
(d) de 0 % en poids à 0,5 % en poids d'un tensioactif non ionique ;
les pourcentages en poids étant basés sur le poids total de la composition

3. Composition d'émulsion selon la revendication 1 ou 2, la composition comprenant :
(a) de 70 % en poids à 90 % en poids d'une phase d'huile ;
(b) de 5 % en poids à 25 % en poids d'un polyol ;
(c) de 0,01 % en poids à 5 % en poids d'un tensioactif amphotère ;
(d) de 0 % en poids à 0,5 % en poids d'un tensioactif non ionique ;
les pourcentages en poids étant basés sur le poids total de la composition

4. Composition d'émulsion selon l'une quelconque des revendications 1 à 3, la composition d'émulsion comprenant moins de 0,1 % poids, plus préférablement moins de 0,01 % en poids, par exemple 0 % en poids de tensioactif non ionique sur la base du poids total de la composition.

5. Composition d'émulsion selon l'une quelconque des revendications 1 à 4, la composition d'émulsion comprenant de 0,01 % en poids à 5 % en poids d'eau sur la base du poids total de la composition.

6. Composition d'émulsion selon l'une quelconque des revendications 1 à 5, le tensioactif amphotère étant choisi dans le groupe constitué par : une bétaïne, un oxyde d'amine, un amphoglycinate et un amphopropionate.

7. Composition d'émulsion selon l'une quelconque des revendications 1 à 6, le tensioactif amphotère étant un composé possédant la formule générale parmi :
**R₁ R₂ R₃ N⁺ (CH₂)_{y} C(O)O⁻** **(I)**
et
**(R₁)(R₂C(O)NH(CH₂)ₓ)(R₃)N⁺(CH₂)_{y} C(O)O⁻** **(II)**
R₁ étant un groupe C₁₋₅-alkyle ou alcényle qui est éventuellement hydroxylé ;
R₂ étant un groupe C₁₀₋₂₂-alkyle ou C₁₀₋₂₂-alcényle ;
R₃ étant indépendamment choisi parmi un groupe C₁₋₅-alkyle ou alcényle, un groupe C₁₀₋₂₂-alkyle ou C₁₀₋₂₂-alcényle ;
x étant un entier de 2 à 4, y étant un entier de 1 à 4.

8. Composition d'émulsion selon l'une quelconque des revendications 1 à 7, le tensioactif amphotère étant un composé possédant la formule générale :
**R₇-N⁺(CH₃)₂-CH₂-COO⁻** **(IX)**
R₇ étant un groupe C₁₀₋₂₂-alkyle ou C₁₀₋₂₂-alcényle.

9. Composition d'émulsion selon la revendication 8, R₇ étant un groupe C₁₆₋₂₂-alkyle ou C₁₆₋₂₂-alcényle.

10. Composition d'émulsion selon l'une quelconque des revendications 1 à 6, le tensioactif amphotère étant un composé possédant la formule générale parmi :
**R₁ R₂ R₃ N⁺R₄-SO₃⁻** **(III)**
et
**(R₁)(R₂C(O)NH(CH₂)ₓ)(R₃)N⁺R₄-SO₃⁻** **(IV)**
R₁ étant un groupe C₁₋₅-alkyle ou alcényle qui est éventuellement hydroxylé ;
R₂ étant un groupe C₁₀₋₂₂-alkyle ou C₁₀₋₂₂-alcényle ;
R₃ étant indépendamment choisi parmi un groupe C₁₋₅-alkyle ou alcényle, un groupe C₁₀₋₂₂-alkyle ou C₁₀-₂₂-alcényle ;
R₄ étant un groupe C₁₋₄-alkyle, linéaire ou ramifié, éventuellement hydroxylé ;
x étant un entier de 2 à 4.

11. Composition d'émulsion selon l'une quelconque des revendications 1 à 6, le tensioactif amphotère étant un composé possédant la formule générale parmi :
**R₁ R₂ R₃ N⁺ --O⁻** **(V)**
et
**(R₁)(R₂C(O)NH(CH₂)ₓ)(R₃)N⁺--O⁻** **(VI)**
R₁ étant un groupe C₁₋₅-alkyle ou alcényle qui est éventuellement hydroxylé ;
R₂ étant un groupe C₁₀₋₂₂-alkyle ou C₁₀₋₂₂-alcényle ;
R₃ étant indépendamment choisi parmi un groupe C₁₋₅-alkyle ou alcényle, un groupe C₁₀₋₂₂-alkyle ou C₁₀₋₂₂-alcényle ;
x étant un entier de 2 à 4.

12. Composition d'émulsion selon l'une quelconque des revendications 1 à 6, le tensioactif amphotère étant un composé possédant la formule générale parmi :
**R₂ N(R₅)(CH₂)_{z} C(O)O⁻ Y⁺** **(VII)**
et
**R₂ C(O)N(R₅)(CH₂)x N(R₆)(CH₂)_{z} C(O)O⁻ Y⁺** **(VIII)**
R₂ étant un groupe C₁₀₋₂₂-alkyle ou C₁₀₋₂₂-alcényle ;
R₅ étant hydrogène ou un groupe en C₁-₅ qui est éventuellement hydroxylé ;
R₆ étant un groupe en C₁₋₅ qui est éventuellement hydroxylé ou un groupe (CH₂)_{z}C(O)O⁻ ;
z étant un entier de 1 à 4 ;
x étant un entier de 2 à 4,
Y⁺ étant un cation.

13. Composition d'émulsion selon l'une quelconque des revendications 1 à 12, le polyol étant un composé possédant la formule :
**R₈-C(-R₉)(-OH)-[A-C(-OH)(R₁₀)]ₙ-R₁₁** **(X)**
R₈, R₉, R₁₀ et R₁₁ étant chacun indépendamment choisis dans le groupe comprenant : hydrogène, fragment C₁₋₆-hydrocarbyle, C₁₋₆-cycloalkyle et C₁₋₆-hétérocyclique ; ledit fragment hydrocarbyle étant saturé ou insaturé, ramifié ou linéaire ; chacun parmi ledit fragment hydrocarbyle, cycloalkyle et hétérocyclique étant éventuellement substitué par un ou plusieurs substituants ;
A étant une liaison covalente ou un fragment de liaison ;
n étant un entier de 1 à 10.

14. Composition d'émulsion selon l'une quelconque des revendications 1 à 13, le polyol étant choisi parmi le propylèneglycol, l'isoprèneglycol, le 1,3-butanediol, le dipropylèneglycol, le glycérol, le diglycérol, le triglycérol, le polyglycérol, le triméthylolpropane, l'érythritol, le pentaérythritol, le sorbitane, le sorbitol, le glucose, le maltitol, le saccharose, le tréhalose, le polyéthylèneglycol et un mélange correspondant.

15. Composition d'émulsion selon l'une quelconque des revendications 1 à 14, la composition d'émulsion comprenant en outre un parfum.
